# EUROPÄISCHE PATENTSCHRIFT

(11) **EP 1 171 347 B1**
(45) Veröffentlichungstag und Bekanntmachung des Hinweises auf die Patenterteilung: **28.01.2004**
(21) Anmeldenummer: 00927019.0
(22) Anmeldetag: 20.04.2000
(51) Int. Cl.: B65B 3/00, A61M 5/30

(54) **VERFAHREN UND VORRICHTUNG ZUM BEFÜLLEN VON NADELFREIEN INJEKTOREN**
METHOD AND DEVICE FOR FILLING NEEDLE-FREE INJECTORS
PROCEDE ET DISPOSITIF DE REMPLISSAGE D'INJECTEURS SANS AIGUILLE

(30) Priorität: 24.04.1999 DE 19918721
(43) Veröffentlichungstag der Anmeldung: 16.01.2002
(73) Patentinhaber: Inova Pharma Systems GmbH, 74523 Schwäbisch Hall (DE)
(72) Erfinder: LAUKENMANN, Bernd, D-74564 Crailsheim (DE); MÜLLER, Michael, Nikolaus, D-74635 Westernach (DE); ROTHBAUER, Jürgen, D-74545 Michelfeld (DE); ARNITZ, Theo Wilfried, 76275 Ettlingen (DE)
(74) Vertreter: Patentanwälte Ruff, Wilhelm, Beier, Dauster & Partner
(86) Internationale Anmeldenummer: PCT/EP2000/003642
(87) Internationale Veröffentlichungsnummer: WO 2000/064744

(56) Entgegenhaltungen:
- WO-A-97/22375
- WO-A-97/36785
- DE-A- 4 320 098
- DE-B- 1 215 541
- FR-A- 889 757
- US-A- 4 338 980

## Beschreibung

Nadelfreie Injektoren, capsules oder dergleichen werden in der Regel von der Seite aus befüllt, von der aus der Kolbenstopfen eingesetzt wird. Es gibt aber auch Fälle, in denen die Befüllung von der Seite der Austrittsöffnung her erfolgen soll, also durch die Austrittsöffnung hindurch. Im folgenden wird für diese Art von Behältnissen nur der Ausdruck Injektoren verwendet, der auch die andere Behältnisse umfassen soll.

Es ist bereits eine nadellose Injektorkapsul in Kombination mit einem zum Füllen der capsule verwendeten Adapter bekannt (WO 97/22375, das eine Vorrichtung gemäß dem Oberbegriff des Anspruchs 1 offenbart). Die capsule bildet eine Kammer mit Injektat und ist mit einer Injektionsöffnung versehen, wobei ein Kolben zur Bewegung innerhalb der Kammer angeordnet ist. Der Adapter ist lösbar mit der capsule verbunden und weist eine mit der capsule in Verbindung stehende Bohrung auf. Die capsule wird dadurch gefüllt, dass unter Vakuum eine Verbindung mit der Füllnadel hergestellt und anschießend das Vakuum wieder abgebaut wird. Die Flüssigkeit wird in die capsule eingedrückt und verschiebt dabei den Kolben.

Der Erfindung liegt die Aufgabe zugrunde, eine Vorrichtung zum exakten Befüllen auch unterschiedlicher Gebinde zu schaffen. Die Gebinde sollen randvoll und zumindest annähernd blasenfrei befüllt werden können.

Zur Lösung dieser Aufgabe schlägt die Erfindung eine Vorrichtung mit den im Anspruch 1 genannten Merkmalen vor. Weiterbildungen der Erfindung sind Gegenstand der abhängigen Ansprüche.

Die von der Erfindung vorgeschlagene Vorrichtung weist eine Halterung für den zu befüllenden Injektor auf, wobei die Halterung vorzugsweise einen Anschlag für den Kolbenstopfen aufweisen kann.

Es kann auch vorgesehen sein, dass die Vakuumglocke an der Füllnadel gehaltert ist, und zwar in einer solchen Weise, dass sie gegenüber der Füllnadel noch bewegt werden kann, vorzugsweise gegen die Wirkung einer Feder. Auf diese Weise wird es möglich, zum Absenken der Vakuumglocke den Antrieb für die Füllnadel zu verwenden.

Die Verschlusseinrichtung kann eine die Füllnadel axial durchsetzende Verschlussstange aufweisen, die durch einen Antrieb bewegt werden kann. Eine derart ausgestaltete Füllnadel hat den Vorteil, dass sie tropfenbildungsfrei schliesst. Durch entsprechende Werkstoffwahl, die demontagefreundliche und totraumarme Konstruktion kann die Füllnadel einfach gereinigt und sterilisiert werden.

Die Vakuumglocke kann eine die Füllnadel umgebende Ansaugöffnung aufweisen.

Damit ist das Evakuieren und das Befüllen in ein gemeinsames Füllorgan integriert.

Bei der Dimensionierung des Systems ist darauf zu achten, dass pharmazeutische Gesichtspunkte berücksichtigt werden. Sterilfilter sind an allen produktberührenden Zu- und Abgängen des Systems vorsehbar.

Weitere Merkmale, Einzelheiten und Vorzüge von Ausbildungen der Erfindung ergeben sich aus der folgenden Beschreibung der Erfindung sowie anhand der Zeichnung. Hierbei zeigen:
- Fig. 1: schematisch die Anordnung einer Vorrichtung zur Saugfüllung nach der Erfindung;
- Fig. 1a: schematisch die Anordnung einer Vorrichtung zur Druckfüllung nach der Erfindung;
- Fig. 2: einen Teil der Vorrichtung nach Fig. 1, nämlich eine Vakuumglocke und eine Füllnadel;
- Fig. 2a: einen Teil der Vorrichtung nach Fig. 1a, nämlich eine Füllnadel;
- Fig. 3: eine Halterung für einen zu befüllenden Injektor;
- Fig. 3a: die Anordnung des Stopfens im Injektor vor der Befüllung;
- Fig. 4: bis 9 die verschiedenen Stadien der Vorrichtung während des Befüllens eines Injektors.

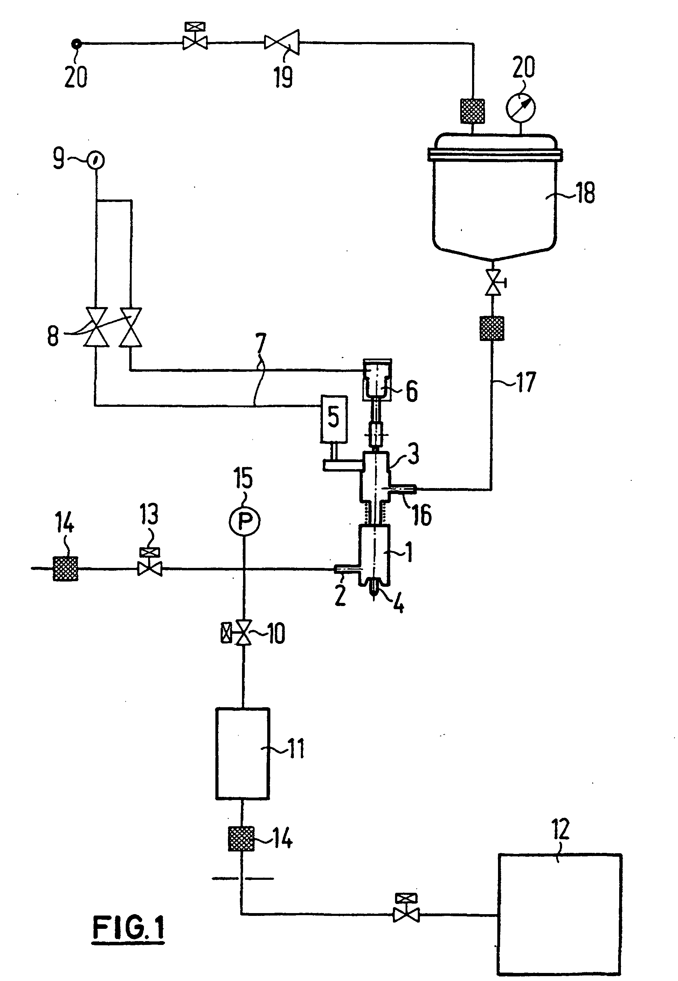

Figur 1 zeigt eine Übersicht über eine Anlage zur Saugfüllung, in der eine Vorrichtung nach der Erfindung angeordnet ist. Die eigentliche Vorrichtung ist in der Mitte der Figur 1 dargestellt. Sie enthält eine Vakuumglocke 1, die mit einem Anschluss 2 für Unterdruck versehen ist. Die Vorrichtung enthält weiterhin eine Befülleinrichtung 3, die eine Füllnadel 4 enthält. Die Füllnadel 4 ragt an der Unterseite der Vakuumglocke 1 aus dieser heraus. Der zu befüllende Injektor, der in der Fig. 1 nicht dargestellt ist, wird unmittelbar unterhalb der Vakuumglocke 1 und der Füllnadel 4 angeordnet. Die Füllnadel 4 und die Vakuumglocke 1 können zusammen durch einen ersten Antrieb 5 abgesenkt und wieder angehoben werden. Dieser Antrieb 5 kann beispielsweise druckluftgesteuert sein. Mit Hilfe eines zweiten koaxialen Antriebs 6 kann eine Verschlusseinrichtung die Füllnadel 4 öffnen und schließen. Zur Ansteuerung dieser beiden Antriebe sind Druckleitungen 7 vorhanden, die über Steuerventile 8 mit einer Druckluftquelle 9 in Verbindung stehen.

Der Anschluss 2 für Unterdruck steht über ein Ventil 10 mit einem Vakuumtank 11 in Verbindung, in dem das Vakuum durch eine Vakuumpumpe 12 aufrechterhalten wird. Der Anschluss 2 kann durch ein weiteres Ventil 13 belüftet werden. In allen Leitungen sind Sterilfilter 14 vorhanden, da es sich bei der abzufüllenden Flüssigkeit in der Regel um medizinische Flüssigkeiten handelt. Der in der zu dem Anschluss 2 führenden Leitung herrschende Druck kann mit Hilfe einer Messeinrichtung 15 gemessen werden.

Die Füllnadel 4 weist einen Anschluss 16 für die einzufüllende Flüssigkeit auf. Dieser Anschluss 16 steht über eine Leitung 17 mit einem Tank 18 in Verbindung, in dem die zu befüllende Flüssigkeit untergebracht ist. Zur Herstellung eines Druckes, der die Flüssigkeit befördert, kann über ein Regelventil 19 eine Verbindung zu einer Druckluftquelle 20 hergestellt werden. Der in dem Tank 18 herrschende Druck kann ebenfalls mit Hilfe eines Messgeräts 20 gemessen werden.

Die verschiedenen Ventile können über eine entsprechende nicht dargestellte Steuerung geöffnet und geschlossen werden.

Die Figur 1a zeigt in schematischer Darstellung die Anordnung einer Vorrichtung zur Druckbefüllung nach der Erfindung. Die Vorrichtung enthält eine Befülleinrichtung 3 mit ihrer Füllnadel 4. Die Füllnadel 4 ragt an der Unterseite von der Befülleinrichtung 3 in Richtung auf den zu befüllenden Injektor, der in der Figur 1a nicht dargestellt ist, ab. Die Befülleinrichtung 3 mit ihrer Füllnadel 4 kann durch den ersten Antrieb 5 abgesenkt und wieder angehoben werden. Dieser Antrieb 5 kann beispielsweise wie dargestellt druckluftgesteuert sein. Dabei gelangt die Vakuumglocke 1 dichtend in Anlage mit der Dichtungshülse 44, wobei der Injektor und der Raum in der bichtungshülse 44 dann evakuierbar ist.
Mit einem zweiten, koaxiallen Antrieb 6 kann eine Verschlusseinrichtung die Füllnadel 4 öffnen und schliessen. Zur Ansteuerung dieser beiden Antriebe sind Druckleitungen 7 vorhanden, die über Steuerventile 8 mit einer Druckluftquelle 9 in Verbindung stehen.

Die Füllnadel 4 weist einen Anschluss 16 für die einzufüllende Flüssigkeit auf. Dieser Anschluss 16 steht über die Leitung 17 mit dem Drucktank 18 in Verbindung. In dem Drucktank 18 ist die zu befüllende Flüssigkeit untergebracht. Zur Erzeugung eines ausreichenden Fülldruckes, also einem Überdruck in dem Drucktank 18 kann über ein Regelventil 19 eine Verbindung zu einer Druckluftquelle 20 hergestellt werden. Damit eine Verunreinigung der Flüssigkeit, die insbesondere eine medizinische Flüssigkeit sein kann, über die zugeführte Druckluft verhindert wird, ist vorzugsweise zwischen dem Regelventil 19 und dem Drucktank 18 seitens der Druckluftzufuhr ein Sterilfilter 14 angeordnet. Darüber hinaus sind beispielsweise elektrisch betätigbare Schaltventile 55 in der Leitung 17 angeordnet. Ebenfalls in der Leitung 17 kann in dem Fluidpfad zwischen Tank 18 und Füllnadel 4 an weiterer Sterilfilter 14 angeordnet sein. Es gibt zusätzlich auch einen Flüssigkeitspfad, der von der Leitung 17 zu einem Flüssigkeitsauffangbehälter 56 führt und über den die Leitung 17 druckentlastet werden kann.

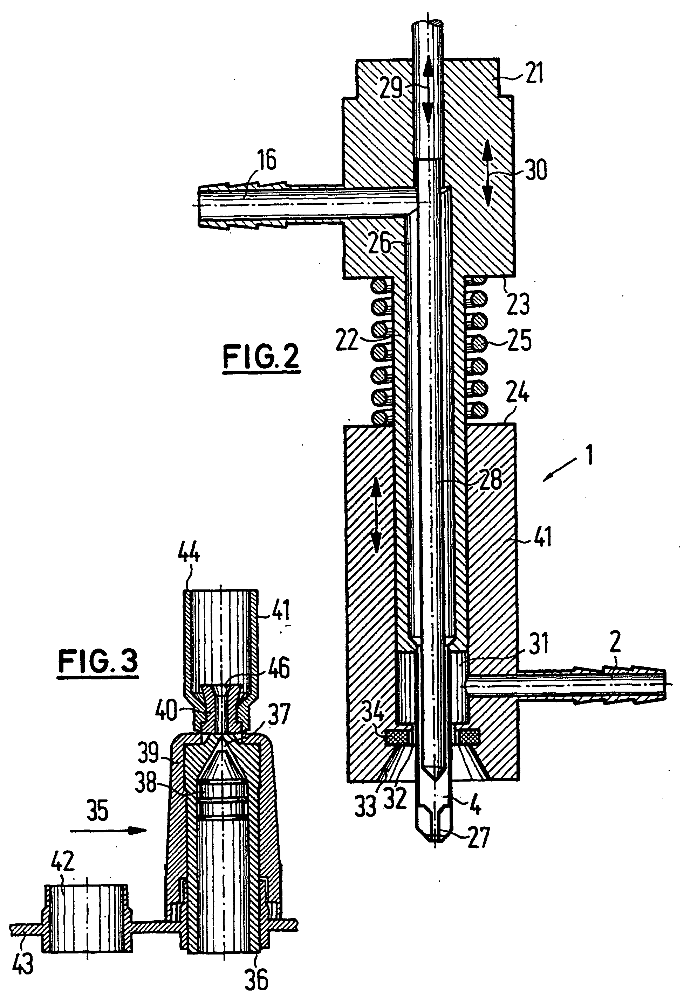

Figur 2 zeigt in größerem Maßstab Einzelheiten der Vakuumglocke 1 und der Füllnadel 4. An einem Bauteil 21, in das der Anschluss 16 für die einzufüllende Flüssigkeit mündet, ist ein hohler zylindrischer Ansatz 22 angebracht, an dessen zylindrischer Außenseite die Vakuumglocke 1 verschiebbar geführt ist. Zwischen einer Schulter 23 des Bauteils 21 und einer dieser gegenüberliegenden Stirnfläche 24 der Vakuumglocke 1 erstreckt sich eine Feder 25. Durch den hohlzylindrischen Ansatz 22 erstreckt sich ein Rohr 26, das in dem Bereich seines freien Endes in die Füllnadel 4 übergeht. Die Füllnadel 4 weist im Bereich ihres freien Endes, unten in Figur 2, eine Öffnung 27 mit einem deutlich kleineren Durchmesser als ihr Außendurchmesser auf. Das Rohr 26 steht mit dem Anschluss 16 in Verbindung.

Durch das Rohr 26 und die Füllnadel 4 erstreckt sich eine Verschlussstange 28, die durch eine seitliche Öffnung im Bereich des oberen Endes des Rohres 26 durch die Wand der Zuleitung für die Flüssigkeit hindurchgeht. Dort erfolgt eine nicht näher dargestellte Abdichtung. Die Stange ist mit dem bereits erwähnten und in Figur 1 dargestellten Antrieb 6 verbunden und kann in Richtung des Doppelpfeils 29 bewegt werden.

Der Antrieb 5 kann das Bauteil 21 und damit die Füllnadel 4 in Richtung des Doppelpfeils 30 bewegen. Bei der Abwärtsbewegung des Bauteils 21 und damit der Füllnadel 4 wird gleichzeitig auch die Vakuumglocke 1 mit bewegt. Erst dann, wenn die Vakuumglocke 1 gegen einen Körper anschlägt, bleibt sie stehen, während die Füllnadel 4 weiter bewegt werden kann.

Der Anschluss 2 für Unterdruck mündet in einem die Füllnadel umgebenden Hohlraum 31, von dem aus durch einem schmalen ringförmigen Spalt 32 Luft aus der Umgebung der Füllnadel von außerhalb der Vakuumglocke angesaugt werden kann. Die Vakuumglocke 1 weist eine diesen Spalt 32 umgebende Kegelstumpffläche 33 auf, an deren dem Spalten 32 zugewandten Endes eine Dichtung 34 angeordnet ist.

Die Figur 2a zeigt in schematischer Darstellung die Füllnadel 4 für eine Befüllung von Injektoren, die kein Dichtsystem zum Abdichten der Schnittstelle Injektor-Nadelspitze besitzen. Dabei wurde die Vakuumglocke 1 nicht dargestellt. Die Füllnadel weist an ihrer Vorderkante, die mit dem Injektor in Anlage gerät, eine Dichtung 57 auf, damit eine druckdichte Anlage an den Injektor ermöglicht wird. Der Anschluss 16 für die Befülleinrichtung 3 mündet in dem Bauteil 21. Von dem Bauteil 21 ragt ein hohler zylindrischer Ansatz 22 in Richtung auf den Injektor ab. Durch den hohlzylindrischen Ansatz 22 erstreckt sich ein Rohr 26, das in dem Bereich seines freien Endes in die Füllnadel 4 übergeht. Die Füllnadel 4 weist im Bereich ihres freien Endes, unten in Figur 2a, eine Öffnung 27 mit einem deutlich kleineren Durchmesser als ihr Aussendurchmesser auf.

Es besteht eine fluidische Verbindung zwischen dem Rohr 26 und dem Anschluss 16. Durch das Rohr 26 und die Füllnadel 4 erstreckt sich eine Verschlusstange 28, die durch eine seitliche Öffnung im Bereich des oberen Ende des Rohres 26 durch die Wand der Zuleitung für die Flüssigkeit hindurchgeht. Dabei ist die Durchtrittstelle der Verschlusstange 28 durch das Rohr 26 druckdicht ausgebildet, so dass keine Leckage von Flüssigkeit entstehen kann. Diese Abdichtung ist nicht näher dargestellt. Die Verschlusstange 28 ist mit dem in Figur 1 dargestellten Antrieb 6 verbunden und kann in Richtung des Doppelpfeils 29 bewegt werden.

Der Antrieb 5 kann das Bauteil 21 und damit die Füllnadel 4 in Richtung des Doppelpfeiles 30, also in Richtung auf den Injektor bzw. von dem Injektor weg, bewegen. Bei der Abwärtsbewegung des Bauteils 21 wird die Füllnadel 4 soweit in Richtung auf den Injektor bewegt, bis die Dichtung 57 dichtend an dem Injektor anliegt.

Die in Figur 2 und Figur 1a dargestellten Einrichtungen arbeiten mit dem in Figur 3 dargestellten Injektor 35 zusammen. Der Injektor enthält einen Glaskörper 36, der zur Aufnahme der Flüssigkeit bestimmt ist und einen zylindrischen Innenraum 37 aufweist. In dem Innenraum ist ein Kolbenstopfen 38 angeordnet, der drei umlaufende Dichtungen aufweist und dadurch in dem Innenraum gehalten ist. An der in Figur 3 oberen Seite geht der zylindrische Innenraum 37 in einen kegelförmigen Raum über, der über eine relativ kleine Austrittsöffnung nach außen führt.

Der Glaskörper 36 mit dem Kolbenstopfen 38 ist in einer äußeren Kunststoffhülse 39 eingesetzt, die eine Halterung für eine zu der Austrittsöffnung führende Dichtungshülse 40 bildet. Auf der dem Glaskolben 36 abgewandten Seite geht die Kunststoffhülse 39 in eine weitere Hülse 41 mit einem etwas kleineren Außendurchmesser über.

Der Injektor 35 steckt in einer Hülse 42 eines Aufnahmenestes 43, das zum Transport und zur Halterung der Injektoren 35 dient. Das Aufnahmenest ist plattenförmig ausgebildet und dient zur Aufnahme mehrerer Injektoren 35.

Die Stirnkante 44 der dem Glaskörper 36 abgewandten Hülse 41 ist so bemessen, dass sie zur Anlage an der Unterseite der Dichtung 34 der Vakuumglocke 1 bestimmt und geeignet ist. Zum Befüllen wird die Vakuumglocke 1 auf die Hülse 41 abgesenkt, bis diese Stirnkante 44 an der Dichtung 34 anliegt. Durch die Dichtungshülse 40 erfolgt das Evakuieren des Innenraumes des Glaskörper 36 zwischen dessen Austrittsöffnung und dem Kolbenstopfen 38. Dann kann die Füllnadel 4 bis in Kontakt mit der Dichtungshülse 40 bewegt werden. Durch die Dichtungshülse 40 erfolgt ebenfalls das Befüllen des Glaskörpers 36.

Die Figur 3a zeigt in nochmals vergrösserter Ausschnittsdarstellung die Gestaltung eines Injektors, der insbesondere zur Verwendung bei einer Druckbefüllung geeignet ist. In dieser Figur ist der Glaskörper 36 dargestellt, in dem der Kolbenstopfen 38 verschiebbar gelagert angeordnet ist. Im Glaskörper wird, verschlossen durch den Kolbenstopfen 38, ist der zylindrische Hohlraum 37 ausgebildet. Zur fluiddichten Anlage der füllnadelseitigen Dichtung 57 weist der Glaskörper 36 eine konische Ausformung 58 auf, was insbesondere bei Druckbefüllungen vorteilhaft ist.

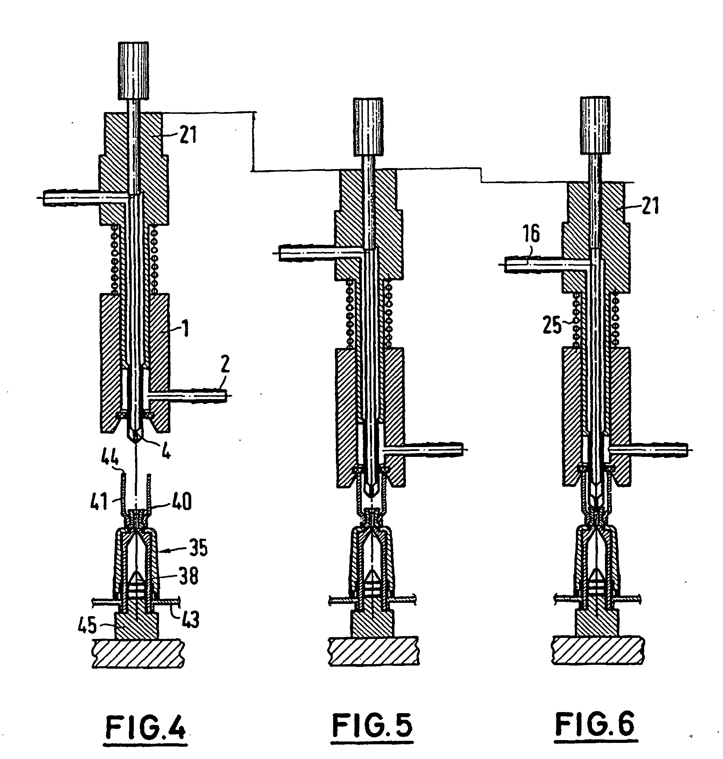

Ein Verfahrensablauf wird nun anhand der Figuren 4 bis 9 dargestellt und beschrieben, die verschiedenen Stadien von mit der Vorrichtung der Erfindung durchführbaren Verfahren darstellen.

Figur 4 zeigt die Ausgangsposition eines möglichen Verfahrens. Der Injektor 35 ist in dem Aufnahmenest 43 gehaltert. Er ist so auf einen Anschlag 45 aufgesetzt, dass der Kolbenstopfen 38 eine bestimmte Position einnimmt, die der gewünschten Sollposition des Kolbenstopfens 38 entspricht. Die Austrittsöffnung und die Dichtungshülse 40 sind axial gegenüber der Füllnadel 4 ausgerichtet. Durch Betätigen des Antriebs 5 wird das Bauteil 21 mit der Füllnadel 4 und der Vakuumglocke 1 abgesenkt. Diese Bewegung wird fortgesetzt, bis die Dichtung 34 auf der Stirnkante 44 so aufliegt, dass die Feder vorgespannt ist. Damit können Höhenunterschiede zwischen den Kapsulen ausgeglichen werden. Dies ist in Figur 5 dargestellt. Nun wird der Anschluss 2 unter Unterdruck gesetzt, so dass die Luft aus dem Innenraum 37 oberhalb des Kolbenstopfens 38 durch den Ringspalt 32 abgesaugt wird. Der abgedichtete Innenraum der Hülse 41 und das Innere des Glaskörpers 36 werden evakuiert. Sobald die Evakuierung abgeschlossen ist, also das Vakuum einen ausreichend hohen Wert erreicht, wird das Bauteil 21 weiter abgesenkt, wodurch die Feder 25 weiter gespannt und die Füllnadel 4 in Kontakt mit der äußeren Öffnung 46 der Dichtungshülse 40 gebracht wird. Dieser Zustand ist in Figur 6 dargestellt. Nun wird durch Betätigen des Antriebs 6 die Verschlussstange 28 angehoben, so dass die Öffnung 27 der Füllnadel 4 geöffnet wird. Auch dies ist in Figur 6 dargestellt. Die Flüssigkeit kann nun durch den Anschluss 16 und die Füllnadel 4 in den evakuierten Innenraum 37 des Glaskörpers 36 strömen. Sobald das Vakuum in diesem Innenraum beseitigt ist, ist der Innenraum befüllt.

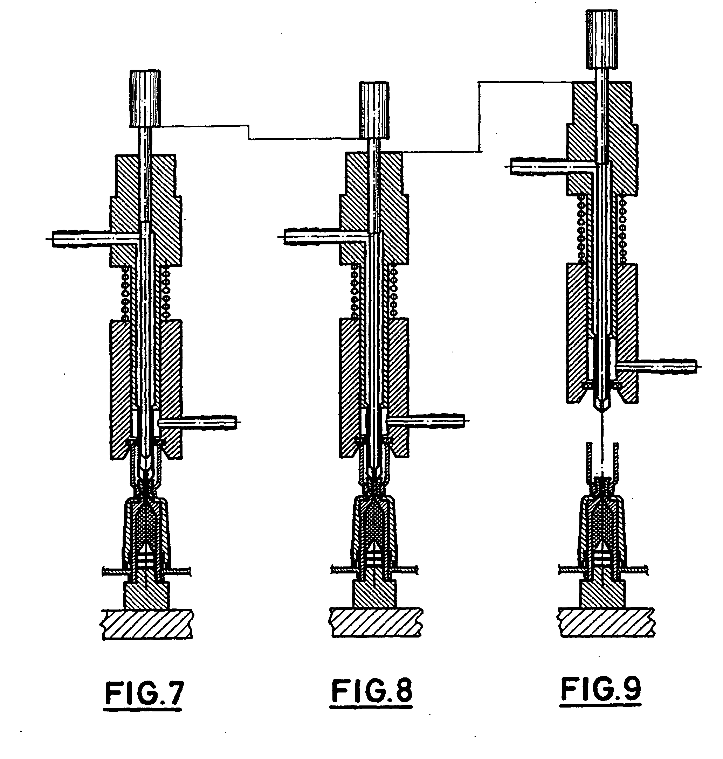

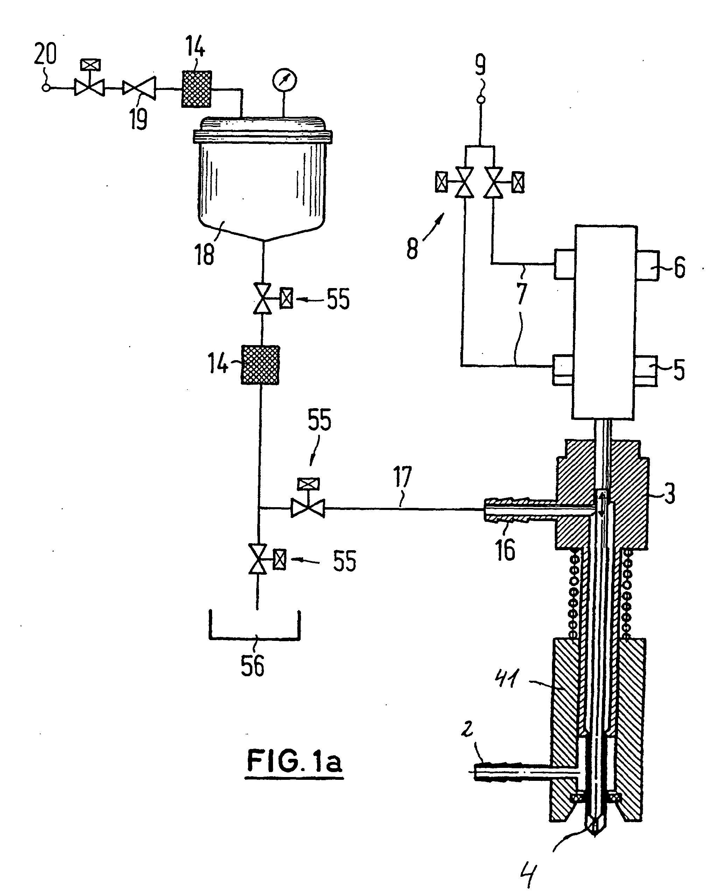

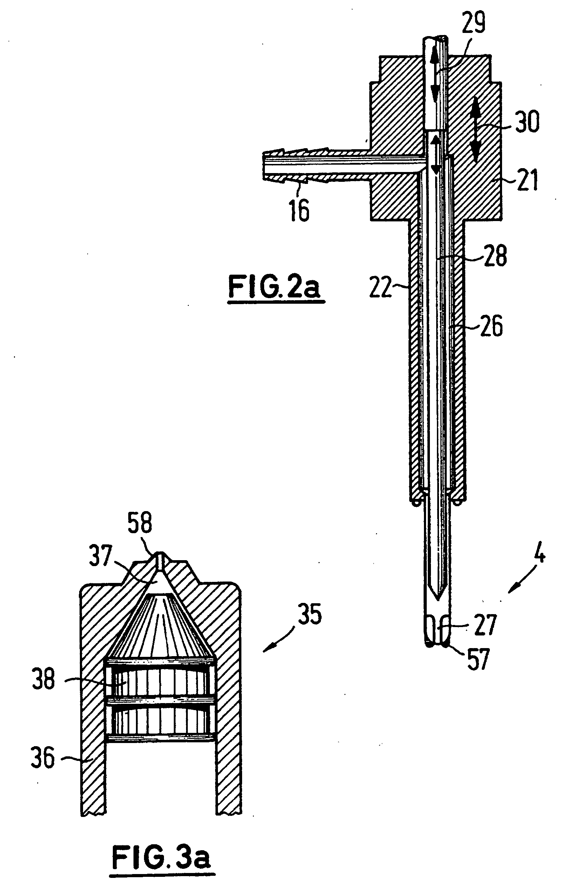

Sobald der Innenraum 37 befüllt ist, siehe Figur 7, wird ein Bypass geöffnet, um einen ggf. vorhandenen Überdruck abzubauen. Die Verschlussstange 28 wird wieder abgesenkt, bis sie die Öffnung 27 verschließt. Dies zeigt die Figur 8. Spätestens zu diesem Zeitpunkt wird der von der Hülse 41 begrenzte Raum wieder über die Vakuumglocke 1 belüftet, damit die Vakuumglocke 1 wieder von dem Injektor 35 abgehoben werden kann. Anschließend wird die Einheit aus Vakuumglocke 1, Bauteil 21 und Füllnadel 4 angehoben, so dass der gefüllte Injektor 35 weitertransportiert werden kann.

Bei dem unter Bezugnahme auf die Figuren 4 bis 9 beschriebenen Verfahren war der Kolbenstopfen 38 in seiner endgültigen Sollposition angeordnet worden, bevor die Evakuierung und Befüllung begann.

Das Verfahren kann auch so gestaltet werden, dass der Kolbenstopfen weiter in den Innenraum eingeschoben wird, bevor mit dem Evakuieren begonnenen wird. Eine solche Ausgangsposition ist in Figur 3 dargestellt. Hier ist es sinnvoll, das Befüllen unter höherem Druck durchzuführen, um den Kolbenstopfen 38 nach außen zu verschieben, bis er beispielsweise an dem Anschlag 45 zur Anlage kommt. Auch in diesem Fall wird der Injektor 35 randvoll und zumindest annähernd blasenfrei befüllt.

## Patentansprüche

1. Vorrichtung zum Befüllen von nadelfreien Injektoren (35) wie capsules oder dergleichen, mit
1.1 einer Halterung für den Injektor (35) welcher Injektor einen eingesetzten Kolbenstopfen (38) aufweist,
1.2 einer Vakuumglocke (1),
1.2.1 deren Innenraum mit dem Innenraum (37) des Injektors (35) von der Seite der Austrittsöffnung her verbindbar ist,
1.3 einer Füllnadel (4), die
1.3.1 mit der Austrittsöffnung des Injektors (35) zu dessen Befüllung durch die Austrittsöffnung verbindbar ist,
1.4 einem Anschluss (16) für die einzufüllende Flüssigkeit und
1.5 einem Anschluss (2) für Unterdruck,
**gekennzeichnet durch**
1.6 eine Verschlusseinrichtung für die Füllnadel (4) zur Beendung der Befüllung bei vollständig befülltem Innenraum (37) des Injektors (35).

2. Vorrichtung nach Anspruch 1, bei der die Halterung für den Injektor (35) einen Anschlag (45) für den Kolbenstopfen (38) auf weist.

3. Vorrichtung nach Anspruch 1 oder 2, bei der die Vakuumglocke (1) an der Füllnadel (4) dieser gegenüber verschiebbar gehaltert und federbeaufschlagt ist.

4. Vorrichtung nach einem der Ansprüche 1 bis 3, bei der die Verschtusseinrichtung eine die Füllnadel (4) axial durchsetzende Verschlussstange (28) mit einem Antrieb (6) auf weist.

5. Vorrichtung nach einem der Ansprüche 1 bis 4, bei der die Vakuumglocke (1) eine die Füllnadel (4) umgebende Ansaugöffnung (32) auf weist.

6. Vorrichtung nach einem der Ansprüche 1 bis 5, bei der die Füllnadel (4) mit einem Dichtsystem zur Abdichtung zwischen Nadelspitze und injektor ausgestattet ist.

## Claims

1. Device for filling needle-free injectors (35) such as capsules or similar items, with
1.1 a retainer for the injector (35), the injector of which has an inserted piston plug (38),
1.2 a vacuum bell jar (1),
1.2.1 the interior space of which may be connected with the interior space (37) of the injector (35) from the side where the outlet is located,
1.3 a filling needle (4), which
1.3.1 may be connected with the outlet of the injector (35) in order to fill the latter via the outlet,
1.4 a connection (16) for the fluid to be filled and
1.5 a connection (2) for negative pressure,
**characterised by**
1.6 a sealing device for the filling needle (4) to terminate the filling process once the interior space (37) of the injector (35) is completely full.

2. Device according to claim 1, whereby the retainer for the injector (35) has a stop (45) for the piston plug (38).

3. Device according to claim 1 or 2, whereby the vacuum bell jar (1) is retained at the filling needle (4), may be displaced vis-à-vis the latter and is springloaded.

4. Device according to one of claims 1 to 3, whereby the sealing device has a sealing rod (28) axially traversing the inside of the filling needle (4) and a drive (6).

5. Device according to one of claims 1 to 4, whereby the vacuum bell jar (1) has a suction opening (32) surrounding the filling needle (4).

6. Device according to one of claims 1 to 5, whereby the filling needle (4) is equipped with a sealing system for sealing between needlepoint and injector.

## Revendications

1. Dispositif pour le remplissage d'injecteurs sans aiguille (35) comme les capsules ou dispositifs de ce type, avec
1.1 un support pour l'injecteur (35) lequel injecteur présente un bouchon de piston rapporté (38),
1.2 une cloche à vide (1),
1.2.1 dont l'espace intérieur peut être relié avec l'espace intérieur (37) de l'injecteur (35) du côté de l'orifice de sortie,
1.3 une aiguille de remplissage (4), qui
1.3.1 peut être reliée avec l'orifice de sortie de l'injecteur (35) pour son remplissage à travers l'orifice de sortie,
1.4 un raccord (16) pour le liquide à remplir et
1.5 un raccord (2) pour le vide,
**caractérisé par**
1.6 un dispositif de fermeture pour l'aiguille de remplissage (4) pour mettre fin au remplissage lorsque l'espace intérieur (37) de l'injecteur (35) est plein.

2. Dispositif selon la revendication 1, dans lequel le support pour l'injecteur (35) présente une butée (45) pour le bouchon de piston (38).

3. Dispositif selon la revendication 1 ou 2, dans lequel la cloche à vide (1) est maintenue sur l'aiguille de remplissage (4) de manière mobile par rapport à celle-ci et commandée par ressort.

4. Dispositif selon l'une des revendications 1 à 3, dans lequel le dispositif de fermeture présente une tige de fermeture (28) traversant axialement l'aiguille de remplissage (4) ainsi qu'un entraînement (6).

5. Dispositif selon l'une des revendications 1 à 4, dans lequel la cloche à vide (1) présente un orifice d'aspiration (32) entourant l'aiguille de remplissage (4).

6. Dispositif selon l'une des revendications 1 à 5, dans lequel l'aiguille de remplissage (4) est munie d'un système d'étanchéité pour l'étanchéité entre la pointe de l'aiguille et l'injecteur.
